# EUROPÄISCHE PATENTSCHRIFT

(11) **EP 1 560 603 B1**
(45) Veröffentlichungstag und Bekanntmachung des Hinweises auf die Patenterteilung: **07.03.2007**
(21) Anmeldenummer: 03776893.4
(22) Anmeldetag: 10.11.2003
(51) Int. Cl.: A61K 51/12, A61K 49/00

(54) **VERFAHREN ZUR MESSUNG DES METABOLISMUS DER BAUCHSPEICHELDRÜSE**
METHOD FOR MEASURING PANCREATIC METABOLISM
PROCEDE DE MESURE DU METABOLISME DU PANCREAS

(30) Priorität: 12.11.2002 DE 10252390
(43) Veröffentlichungstag der Anmeldung: 10.08.2005
(73) Patentinhaber: Infai Institut für Biomedizinische Analytik und NMR-Imaging GMBH, 44799 Bochum (DE)
(72) Erfinder: AYGEN, Sitke, INFAI Inst. für biomed. Analyzik Und, 51105 Köln (DE)
(74) Vertreter: Schreiber, Christoph
(86) Internationale Anmeldenummer: PCT/EP2003/012515
(87) Internationale Veröffentlichungsnummer: WO 2004/043498

(56) Entgegenhaltungen:
- AYGEN S ET AL: "Diagnostic value of MTG-BT for the diagnosis of exocrine pancreatic insufficiency in comparison to secretin pancreozymin test" KLINICKA BIOCHEMIE A METABOLISMUS 1997 CZECH REPUBLIC, Bd. 5, Nr. SUPPL., 1997, Seiten 19-20, XP0008027719 ISSN: 1210-7921
- LÖSER C ET AL: "Comparative clinical evaluation of the 13C-mixed triglyceride breath test as an indirect pancreatic function test." SCANDINAVIAN JOURNAL OF GASTROENTEROLOGY. MAR 1998, Bd. 33, Nr. 3, März 1998 (1998-03), Seiten 327-334, XP0008027732 ISSN: 0036-5521 in der Anmeldung erwähnt
- GHOOS Y F ET AL: "A mixed-triglyceride breath test for intraluminal fat digestive activity." DIGESTION. 1981, Bd. 22, Nr. 5, 1981, Seiten 239-247, XP0008027842 ISSN: 0012-2823

## Beschreibung

Gegenstand der vorliegenden Erfindung ist ein Verfahren zur Messung des Metabolismus der Bauchspeicheldrüse bei exokriner Pankreaseinsuffizienz (EPI) leichten, mittelschweren .und schweren Grades.

Exokrine Pankreaseinsuffizienz ist gekennzeichnet durch eine Verminderung der Abgabe von Enzymen aus dem Pankreas, insbesondere von Amylase, Lipase und Chymotrypsin sowie die Verminderung der Freisetzung von Bicarbonat.

Der bisherige sogenannte "Goldstandard" der Bestimmung von EPI besteht darin, dass über einen Zeitraum von zwei Stunden Sekretin und in der zweiten Stunde zusätzlich Caerulein intravenös infundiert wird und dabei über eine Duodenalsonde das Duodenalsekret abgesaugt und analysiert wird. Diese Bestimmungsmethode ist aufwendig, teuer und für den Patienten sehr unangenehm und obendrein störanfällig. Viele Patienten reagieren zudem allergisch auf Caerulein. Außerdem ist der Test störanfällig und deshalb mit einer erheblichen Fehlerquote behaftet. Fehlt eine standardisierte Testdurchführung, ist die Auswertung abhängig von den im jeweiligen Labor erhobenen Referenzwerten (siehe J.-Matthias Löhr: Exokrine Pankreaseinsuffizienz, 1. Auflage, Bremen: Uni-Med, 2001).

Ein einfacherer, aber ebenfalls nicht sehr genauer Test besteht in der Bestimmung der Elastase im Stuhl.

Ein Pankreasfunktionstest, bei dem vor allem die Amylase bestimmt wird, ist in der DE-C-44 26 204 beschrieben. Hierbei wird natürliche Maisstärke appliziert und nach Metabolisierung als ¹³C-angereichertes Kohlendioxid über die Atemluft ausgeschieden und analysiert. Der Test beruht darauf, dass Maisstärke von Natur aus mit ¹³C-Atomen angereichert ist.

Ein Nachteil des Testes besteht darin, dass hiermit nur die exokrine Pankreaseinsuffizienz schweren Grades bestimmt werden kann. Dies liegt unter anderem daran, dass die Bauchspeicheldrüse nicht die alleinige Quelle von Amylase ist und der Test daher durch Amylase im Speichel- und Magensekret gestört wird, vgl. Löser et al., Z Gastroenterol 1997; 35: 187-194).

Weitere Untersuchungen der Funktionsfähigkeit der Bauchspeicheldrüse sind entwickelt worden mit ¹⁴C- und ¹³C-angereicherten Substanzen, wobei die ¹³C-Substanzen bevorzugt werden, da sie den Patienten nicht mit dem radioaktiven ¹⁴C-Isotop belasten. Als ¹³C-angereicherte Testsubstanzen sind vor allem Cholesterolester und Triglyceride untersucht worden, wobei insbesondere das gemischte Triglycerid Glyceryl-1,3-dioctadecanoat-2-octanoat-1-¹³C (auch 1,3-Distearyl,2(carboxyl- ¹³C)octanoyl-glycerol genannt) untersucht wurde, da die pankreatische Lipase zunächst die beiden Stearyl-Gruppen hydrolisiert und erst dann die kürzere ¹³C-Octanoyl-Gruppe der Metabolisierung zugeführt wird.

Vergleiche der Untersuchungen mit dem "Goldstandard" und anderen Methoden haben ergeben, dass auch diese Methode nur geeignet ist, pankreatische Insuffizienz schweren und im gewissen Ausmaß mittelschweren Grades zu diagnostizieren. Bei der exokrinen Pankreaseinsuffizienz leichten Grades versagt diese Methode, (vgl. Löser et al., Scand. J Gastroenterol 1998; 33:327-334).

Es besteht somit noch immer das Bedürfnis nach einem einfacheren und auch für den Patienten angenehmeren Bestimmungsverfahren der Funktionsfähigkeit der Bauchspeicheldrüse, insbesondere in Fällen der exokrinen Pankreaseinsuffizienz leichten und mittelschweren Grades durch Messung des Metabolismus der Bauchspeicheldrüse. Die Einstufung wird folgendermaßen vorgenommen:

### Leichte Pankreaseinsuffizienz:

Verminderung des Bicarbonats um < 50%
   oder
Verminderung eines Enzyms um < 50%
   oder
Verminderung von zwei Enzymen um < 25%
   oder
Verminderung eines Enzyms und Bicarbonats um < 25%

### Mittlere Pankreaseinsuffizienz:

Verminderung von mehr als zwei Enzymen und/oder Bicarbonats um 25 - 75%

### Schwere Pankreaseinsuffizienz:

Reduktion aller Enzyme und/oder Bicarbonatsekretion um > 75%
(Lankisch et al., Dig.Dis.Sci. 1983, 28: 490-493)

Alle Berechnungen sind auf die unteren Grenzwerte (Mittelwert -2s) eines Normalkollektives bezogen.

Es wurde jetzt gefunden, dass die Messung dadurch möglich ist, dass vor und nach intravenöser Applikation von Sekretin und vor und nach oraler Applikation eines ¹³C-Mischtriglycerids der Anstieg von ¹³CO₂ in der ausgeatmeten Atemluft bestimmt wird.

Zur Bestimmung des Anstiegs von ¹³CO₂ in der ausgeatmeten Atemluft wird bevorzugt das Verhältnis zwischen ¹³CO₂ und ¹²CO₂ gemessen. Bevorzugt wird zur Messung IRMS eingesetzt.

IRMS (Isotopic Ratio Mass Spectrometry) ist inzwischen die empfindlichste und beste Methode zur Bestimmung von ¹³CO₂ in der ausgeatmeten Atemluft.

Eine alternative Möglichkeit ist die Messung mittels NDIR (Non-Dispersive Infrared Spectroscopy).

Während nach einem Verfahren des Standes der Technik wie z.B. im oben genannten Löser et al. (1998) beschrieben lediglich das ¹³C angereicherte Mischtriglycerid verabreicht wird und anschließend der Anteil an ¹³CO₂ in der Atemluft gemessen wird, ist das erfindungsgemäße Verfahren dadurch gekennzeichnet, dass die Messung unter dem Einfluss einer vorherigen Sekretinapplikation erfolgt.

Dabei zeigt eine verringerte Metabolisierung durch eine verzögerte oder reduzierte Freisetzung von ¹³CO₂ gegenüber gesunden Probanden einen Mangel an Pankreasenzymen und erlaubt damit eine zuverlässige Diagnose.

Beim erfindungsgemäßen Verfahren wird vor Beginn des Tests eine Atemprobe entnommen und dann werden weitere Proben in Zeitintervallen von beispielsweise 15, 20 oder 30 Minuten genommen. Üblicherweise erfolgt die Messung für einem Zeitraum von zwei bis fünf Stunden, typischerweise vier Stunden.

Bevorzugt wird zunächst mit der Applikation des Sekretins begonnen, während der Applikation oder direkt nach Ende der Applikation wird dann das ¹³C angereicherte gemischte Mischtriglycerid verabreicht.

Die ausgeatmete Atemluft kann in Probencontainem aufbewahrt werden und nach Abschluss des Tests gemessen werden. Dabei ist auch ein Transport der Atemluftbehälter möglich.

Als Mischtriglycerid mit ¹³C-Markierung werden bevorzugt solche eingesetzt, bei denen die Säure in der 2 Position die ¹³C-Markierungen trägt. Typischerweise tragen beim erfindungsgemäß eingesetzten Triglycerid die 1 und die 3 Position typische Fettsäuren mit 12 bis 20 C-Atomen, die auch Doppelbindungen enthalten können. In der 2 Position des Triglycerids befindet sich eine kürzere Säure mit 2 bis 12 C-Atomen. Ein besonders bevorzugtes Mischtriglycerid ist das Glyceryl-1,3-dioctadecanoat-2-octanoat-1-¹³C, ein gemischtes Triglycerid, das beispielsweise von der Fa. Sigma-Aldrich in hoher Anreicherung verfügbar ist.

Im Gegensatz zum "Goldstandard", bei welchem Sekretin und anschließend simultan Caerulein im Laufe von zwei Stunden infundiert werden müssen, wird beim erfindungsgemäßen Verfahren nur Sekretin appliziert und dies innerhalb kurzer Zeit , bevorzugt 5 bis 25 Minuten, typischerweise 15 Minuten. Die orale Applikation des ¹³C-Triglycerids erfolgt auch beim erfindungsgemäßen Verfahren mit Hilfe einer standardisierten Testmahlzeit. Anstelle der bisher verwendeten Testmahlzeit aus 250 mg Mischtriglycerids homogenisiert mit 10 g Schokoladencreme in einem 60°C warmen Wasserbad, und Auftragen nach dem Abkühlen auf ein Stück Toast mit 15 g Butter, kann dieses Mischtriglycerid bevorzugt auch einfacher appliziert werden, indem man nur 200 mg Mischtriglycerid beispielsweise mit 15 g Maisstärke, 3 g Kakaopulver und 15 g Butter in 20 ml warmer Milch, um eine homogene Mischung zu erhalten, verrührt und dann auf eine Scheibe Toast streicht. Entscheidend ist nicht die Menge an Fett und Öl, sondern dass das Triglycerid mit den übrigen Komponenten leicht homogen vermischt und auf eine Scheibe Toast aufgetragen werden kann. Prinzipiell besteht auch die Möglichkeit, das Mischtriglycerids in fertig abgepackte Portionen mit stabilen anderen, ausreichend fetthaltigen Komponenten zur Verfügung zu stellen, die dann nur noch auf die Scheibe Toast gestrichen werden müssen.

Beim Vergleich der Testergebnisse des erfindungsgemäßen Verfahrens mit Testergebnissen des "Goldstandards" hat sich zunächst gezeigt, dass die Übereinstimmung zwar ausreichend ist, jedoch noch immer nicht voll befriedigt. Weitere Untersuchungen haben dann ergeben, dass die Reproduzierbarkeit des erfindungsgemäßen Verfahrens sehr hoch ist und die Testergebnisse somit sehr viel enger beieinander liegen als der Vergleich mit dem "Goldstandard" erwarten ließ. Bei der Überprüfung dieses erstaunlichen Ergebnisses wurde festgestellt, dass der "Goldstandard" offensichtlich wegen seiner Aufwendigkeit und Unzumutbarkeit für den Patienten nie auf Reproduzierbarkeit untersucht wurde und daher die Abweichungen von den Ergebnissen des erfindungsgemäßen Verfahrens nicht auf das erfindungsgemäße Verfahren, sondern auf Fehlerquellen beim "Goldstandard" zurückzuführen sind (vgl. Exokrine Pankreaseinsuffizienz 2001, Uni-Med Verlag AG, Bremen, Löhr J.-M. und Schneider H.T.).

Das erfindungsgemäße Verfahren ist somit in vieler Hinsicht dem "Goldstandard" überlegen: es wird auf das Legen der Duodenalsonde verzichtet, was für die Patienten außerordentlich unangenehm ist. Es wird auf die zweistündige Infundierung von Sekretin und Caerulein verzichtet. Stattdessen erfolgt nur eine intravenöse Applikation innerhalb von z.B. ca. 15 Minuten und nur von Sekretin. Da es Patienten gibt, die zwar Sekretin vertragen aber nicht Caerulein, gibt es wesentlich weniger Untersuchungsabbrüche wegen Unverträglichkeit.

Die Kosten für den Test liegen bei ca. einem Zehntel der Kosten des "Goldstandards". Es ist erstmals möglich, auch leichte Grade der exokrinen Pankreaseinsuffizienz zu bestimmen und Patienten mit dieser Diagnose zu therapieren. Auch die exokrine Pankreaseinsuffizienz mittelschweren Grades ist oft noch heilbar oder zumindest stabilisierbar. Pankreatische Insuffizienz schweren Grades ist im allgemeinen nicht mehr therapierbar, sondern nur noch bezüglich der Symptome behandelbar. Von besonderer Bedeutung ist die rechtzeitige Erkennung der Form der pankreatischen Insuffizienz leichten und mittelschweren Grades bei Kindern, zumal bei Kindern die Therapierbarkeit besser und leichter ist als bei Erwachsenen.

Somit eignet sich dieses einfache Verfahren auch als Verlaufskontrolle einer Enzymsubstitutionstherapie.

Erfindungsgemäß wird ein Verfahren zur Bestimmung der Funktionsfähigkeit der Bauchspeicheldrüse, nämlicher milder und mittlerer Form der exokrinen Pankreasinsuffizient bereitgestellt.

### Beispiel

### ¹³C-Atemtest mit Stimulation von Sekretin

Vor Anwendung des ¹³C-Mischtriglycerid-Atemtests sollte der Patient für 6 Stunden (vorzugsweise über Nacht) gefastet haben. Der Test sollte in Ruhelage (sitzend oder liegend) durchgeführt werden.

Der Test beginnt mit Abnahme der ersten Nullwert-Atemprobe ("Nullwert"). Dazu bläst der Patient mit Hilfe eines Strohhalms in das mit "Nullwert" beschriftete Probengefäß. Danach wird dem Patienten 1 U Sekretin/kg Körpergewicht über 15 min intravenös appliziert.

Sekretin ist beispielsweise als Secrelux^{®} von der Firma Goldham Pharma GmbH, Deutschland erhältlich. Eine KE (Klinische Einheit) entspricht 1 U (Unit).

Der Patient nimmt nun innerhalb von 10 Minuten die zubereitete Testmahlzeit zu sich.

Während der nächsten vier Stunden nach Beenden der Testmahlzeit werden zu den im Protokoll festgelegten Zeitabschnitten Atemproben auf die oben beschriebenen Weise in die jeweils eindeutig gekennzeichneten Probengefäße abgegeben.

| Probenentnahme: | | | | | | | | | | | | | | |
|---|---|---|---|---|---|---|---|---|---|---|---|---|---|---|
| 00 | 15 min | 10min | 15 | 30 | 50 | 70 | 90 | 110 | 130 | 150 | 170 | 190 | 210 | 230 |
| min | Sekretin iv | Testmahlzeit | min | min | min | min | min | min | min | min | min | min | min | min |

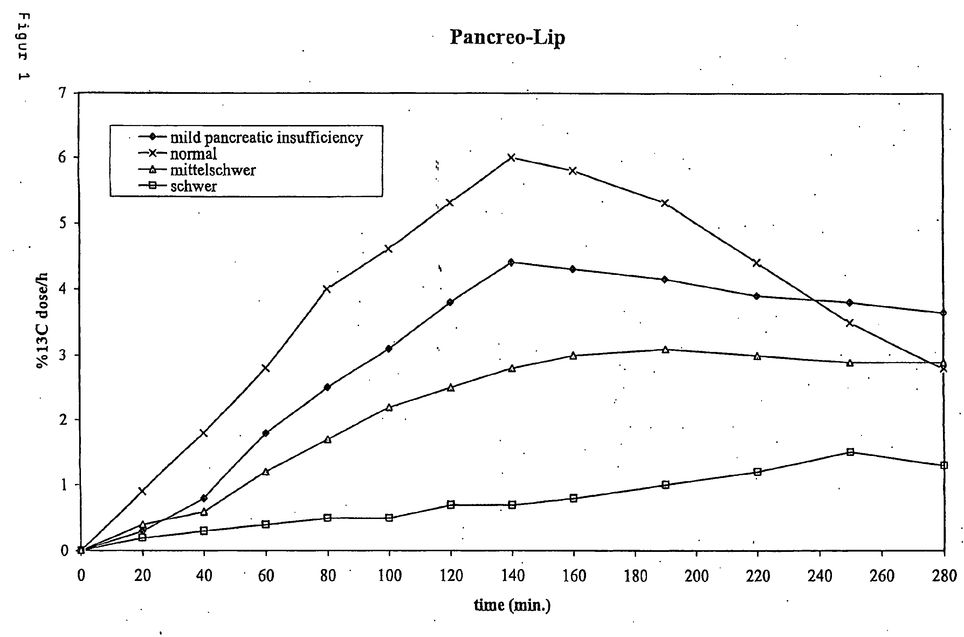

Figur 1 zeigt die Ergebnisse der ¹³CO₂-Atemluftwerte für einen gesunden Probanden (×) und einen Probanden mit EPI, leichten(◆), mittelschweren (▲) und schweren (□) Grades.

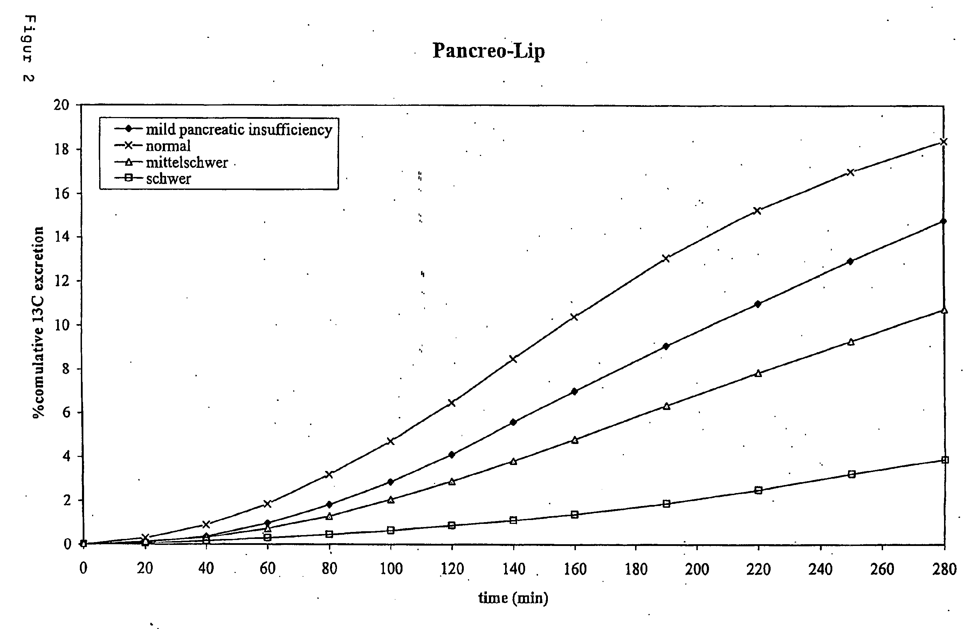

Figur 2 zeigt die kumulativen ¹³CO₂ -Atemluftwerte für einen gesunden Probanden (×) und einen Probanden mit EPI. (**◆**), leichten (◆), mittelschweren (▲) und schweren (□) Grades.

## Patentansprüche

1. Verfahren zur Messung des Metabolismus der Bauchspeicheldrüse, **dadurch gekennzeichnet, dass** vor und nach intravenöser Applikation von Sekretin und vpr und nach oraler Applikation eines ¹³C-Triglycerids der Anstieg von ¹³CO₂ in der ausgeatmeten Atemluft bestimmt wird.

2. Verfahren nach Anspruch 1, **dadurch gekennzeichnet, dass** das ¹³C-Triglycerid das Mischtriglycerid Glyceryl-1,3-dioctadecanoat-2-octanoat-1-¹³C ist.

3. Verfahren nach Anspruch 1 oder 2 **dadurch gekennzeichnet, dass** die Bestimmung von ¹³CO₂ mittels IRMS oder NDIR erfolgt.

4. Verfahren gemäß mindestens einen der Ansprüche 1 bis 3, **dadurch gekennzeichnet, dass** innerhalb von ca. 15 - 30 Minuten 1 U Sekretin/Kg Körpergewicht intravenös appliziert wird.

5. Verfahren gemäß einem der Ansprüche 1 bis 4, **dadurch gekennzeichnet, dass** 200 mg des gemischten Triglycerids mit einer Testmahlzeit appliziert wird.

6. Testkit enthaltend
• Testmahlzeit aus Maisstärke und Kakaopulver
• ¹³C-Mischtriglycerid
• Probenbehälter
• Gebrauchsinformation betreffend ein Verfahren gemäß den Ansprüchen 1 bis 5.

7. Testkit nach Anspruch 6, **dadurch gekennzeichnet, dass** das ¹³C-Mischtrigiycerid Glyceryl-1,3-dioctadecanoat-2-octanoat-1-¹³C ist.

## Claims

1. A method for measuring the metabolism of the pancreas, **characterized in that** the increase of ¹³CO₂ in the exhaled air is determined before and after intravenous administration of secretin and before and after oral administration of a ¹³C-triglyceride.

2. The method according to claim 1, **characterized in that** said ¹³C-triglyceride is the mixed triglyceride glyceryl-1,3-dioctadecanoate-2-octanoate-1-¹³C.

3. The method according to claim 1 or 2, **characterized in that** the determination of ¹³CO₂ is effected by IRMS or NDIR.

4. The method according to at least one of claims 1 to 3, **characterized in that** 1 U of secretin per kg of body weight is administered intravenously within about 15 to 30 minutes.

5. The method according to any of claims 1 to 4, **characterized in that** 200 mg of the mixed triglyceride is administered with a test meal.

6. A test kit containing:
• a test meal of corn starch and cocoa powder;
• a ¹³C-mixed triglyceride;
• a sample container;
• instructions for use relating to a method according to claims 1 to 5.

7. A test kit according to claim 6, **characterized in that** said ¹³C-mixed triglyceride is glyceryl-1,3-dioctadecanoate-2-octanoate-1-¹³C.

## Revendications

1. Procédé de mesure du métabolisme du pancréas, **caractérisé en ce que** l'augmentation de ¹³CO₂ dans l'air expiré est déterminée avant et après une application par voie intraveineuse de sécrétine et avant et après une application par voie orale d'un ¹³C-triglycéride.

2. Procédé selon la revendication 1, **caractérisé en ce que** le ¹³C-triglycéride est le triglycéride mixte glycéryl-1,3-dioctadécanoate-2-octanoate-1-¹³C.

3. Procédé selon la revendication 1 ou 2, **caractérisé en ce que** la détermination de ¹³CO₂ est réalisée par SMRI ou NDIR.

4. Procédé selon au moins l'une quelconque des revendications 1 à 3, **caractérisé en ce que** 1 U de sécrétine/kg de masse corporelle est appliquée par voie intraveineuse en environ 15 à 30 minutes.

5. Procédé selon l'une quelconque des revendications 1 à 4, **caractérisé en ce que** 200 mg de triglycéride mixte sont appliqués avec un repas d'épreuve.

6. Trousse d'essai, contenant :
- un repas d'épreuve composé d'amidon de maïs et de poudre de cacao
- un ¹³C-triglycéride mixte
- un récipient à échantillons
- une notice explicative concernant le procédé selon les revendications 1 à 5.

7. Trousse d'essai selon la revendication 6, **caractérisé en ce que** le ¹³C-triglycéride mixte est le glycéryl-1,3-dioctadécanoate-2-octanoate-1-¹³C.
